(19) Europäisches Patentamt
European Patent Office
Office européen des brevets

(11) Publication number: 0 166 580
A2

(12) # EUROPEAN PATENT APPLICATION

(21) Application number: 85304429.5

(22) Date of filing: 20.06.85

(51) Int. Cl.⁴: C 07 D 501/46

(30) Priority: 25.06.84 US 623865

(43) Date of publication of application: 02.01.86 Bulletin 86/1

(84) Designated Contracting States: AT BE CH DE FR GB IT LI LU NL SE

(71) Applicant: ELI LILLY AND COMPANY, Lilly Corporate Center, Indianapolis Indiana 46285 (US)

(72) Inventor: Heath, Perry Clark, 7130 South Delaware, Indianapolis Indiana 46227 (US)

(74) Representative: Hudson, Christopher Mark et al, Erl Wood Manor, Windlesham Surrey GU20 6PH (GB)

(54) **Improvements in or relating to ceftazidime.**

(57) Crystalline (6R,7R)-7-[(Z)-2-(2-tritylaminothiazol-4-yl)-2-(2-t-butyloxycarbonylprop-2-oxyimino)-acetamido]-3-(1-pyridiniummethyl)-3-cephem-4-carboxylic acid dihydrobromide salt, intermediate to the antibiotic ceftazidime, is provided.

EP 0 166 580 A2

# IMPROVEMENTS IN OR RELATING TO CEFTAZIDIME

This invention relates to an intermediate useful in the preparation of the semi-synthetic cephalosporin antibiotic, ceftazidime. In particular, it relates to a stable crystalline dihydrobromide salt of an amino-protected and carboxy-protected ceftazidime intermediate. The invention further relates to a process for converting the salt intermediate to ceftazidime.

The antibiotic ceftazidime has the chemical name (6R,7R)-7-[(Z)-2-(2-aminothiazol-4-yl)-2-(2-carboxyprop-2-oxyimino)acetamido]-3-(1-pyridinium-methyl)-3-cephem-4-carboxylate and is described in U.S. Pat. No. 4,258,041. Ceftazidime can be prepared by the acylation of the 7-amino-3-(1-pyridiniummethyl)-3-cephem-4-carboxylate nucleus with an amino-protected and carboxy-protected 2-(2-aminothiazol-4-yl)-2-(2-carboxy-prop-2-oxyimino)acetic acid. The trityl group and the t-butyl group are expecially useful protecting groups for the amino and carboxy groups, respectively. The product of the acylation in which these protecting groups are used is (6R,7R)-7-[(Z)-2-(2-tritylaminothiazol-4-yl)-2-(2-t-butyloxycarbonylprop-2-oxyimino)acetamido]-3-(1-pyridiniummethyl)-3-cephem-4-carboxylate. This ceftazidime intermediate is referred to, for convenience, as "blocked intermediate". The blocked intermediate can be isolated directly from the acylation mixture as a crystalline dihydrobromide salt.

In particular, there is provided a process for preparing crystalline (6R,7R)-7-[(Z)-2-(2-tritylaminothiazol-4-yl)-2-(t-butyloxycarbonylprop-2-oxyimino)acetamido]-3-(1-pyridiniummethyl)-3-cephem-4-carboxylic acid dihydrobromide which comprises adding in the presence of a counter solvent, aqueous hydrobromic acid to (6R,7R)-7-[(Z)-2-(2-tritylaminothiazol-4-yl)-2-(2-t-butyloxycarbonylprop-2-oxyimino)acetamido]-3-(1-pyridiniummethyl)-3-cephem-4-carboxylate.

More particularly, ceftazidime blocked intermediate is isolated as a crystalline dihydrobromide salt from the crude acylation reaction mixture in which it is prepared by addition of aqueous hydrobromic acid. Preferably a counter solvent is added to the mixture to enhance the yield of crystalline salt. Unexpectedly, during the formation of the diacid salt, the blocked intermediate does not undergo deblocking to any significant degree.

The crystalline dihydrobromide salt of this invention is represented by formula (I):

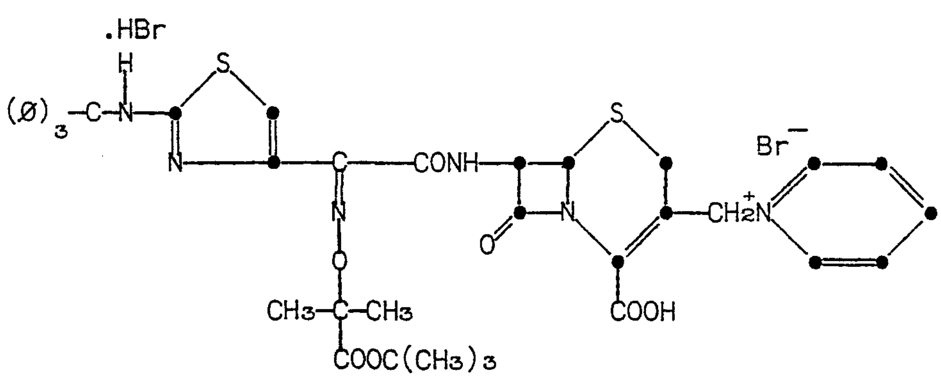

(I)

The crystalline dihydrobromide salt of formula (I) can be obtained directly from the reaction mixture in which the blocked ceftazidime is prepared. The blocked ceftazidine is prepared by the acylation of 7-amino-3-(1-pyridiniummethyl)-3-cephem-4-carboxylate with 2-(2-tritylaminothiazol-4-yl)-2-(2-t-butyloxycarbonylprop-2-oxyimino)acetyl chloride in the presence of an acid scavenger such as triethylamine. The acylation is carried out in an inert solvent, e.g. a halogenated hydrocarbon such as methylene chloride, preferably at a temperature of about 0 to 10°C or lower. Following the completion of the acylation, the reaction mixture is washed with water, preferably containing HBr, and the organic layer is diluted with a counter solvent. An excess of aqueous 48% hydrobromic acid is added to the diluted mixture with agitation. The "counter" solvent can be added to the mixture after the addition of the acid or, preferably, prior thereto. The counter solvent enhances the crystallization of the dihydrobromide salt from the mixture.

The term "counter" solvent refers to an organic solvent in which the dihydrobromide salt is essentially insoluble and one which is miscible with the inert solvent used in the acylation mixture. Examples of counter solvents which can be used are acetone, methyl ethyl ketone, acetonitrile, and isopropyl acetate. The volume of counter solvent used is at least equal to the volume of the acylation mixture following the acidic water wash and, preferably, is 2 to 4 times the volume of the mixture. A preferred counter solvent is methyl ethyl ketone. The choice of counter solvent can influence

the nature of the crystalline form of the di-salt obtained. For example, when acetone and methyl ethyl ketone are used, the crystalline blocked ceftazidime dihydrobromide salt monohydrate is precipitated from the mixture.

The 48% hydrobromic acid is used in an amount greater than the stoichiometric amount needed to form the dihydrobromide salt. Hydrogen bromide (gas) or hydrogen bromide-acetic acid solutions of various concentrations have not produced crystalline precipitates of the salt.

The crystallization of the dihydrobromide salt can be performed at room temperature, athough chilling the mixture to from about -10°C to about 15°C may be beneficial to crystallization and enhanced yields.

Previously, the blocked ceftazidime obtained in the acylation has been isolated as a solvate formed with dimethylacetamide (DMAC). When the acylation is carried out in the presence of excess DMAC, the blocked ceftazidime can be isolated from the acylation mixture as the DMAC solvate. The crystalline dihydrobromide salt of this invention can also be isolated from an acylation reaction mixture containing DMAC. Because the dihydrobromide salt is substantially soluble in DMAC, however, the acylation reaction mixture is washed throroughly with water prior to the addition of hydrobromic acid.

The crystalline salt is separated from the mother liquor by filtration, centrifugation, decantation or by other suitable methods and is washed with a "counter" solvent. The washed salt may be dried if necessary, e.g., in a vacuum oven at or about room temperature.

The crystalline salt of this invention is a stable and convenient form of blocked ceftazidime for storage in bulk. The crystalline salt can be converted readily to the crystalline ceftazidime dihydrobromide salt by treatment with formic acid. For this conversion the dihydrobromide salt is treated with excess 98% formic acid at or somewhat below room temperature, e.g., from 10°C to 20°C. For best results the formic acid is augmented with some 48% hydrobromic acid. The reaction is agitated, for example, by stirring and can be followed by analytical HPLC. After the deblocking reaction is complete, the acidic mixture is cooled to a temperature of about 10°C and is diluted with a large excess of an organic solvent in which the product, ceftazidime dihydrobromide, is substantially insoluble. Organic solvents whcih can be used are acetone, methyl ethyl ketone, acetonitrile, cyclohexanone, or n-propyl alcohol. Upon dilution the crystalline dihydrobromide salt of ceftazidime precipitates and is separated from the mixture by filtration or centrifugation. The salt is washed, preferably with the chilled or cold organic solvent used in the crystallization, and is dried at about room temperature.

The deblocked ceftazidine dihydrobromide salt is converted to crystalline ceftazidime pentahydrate by known procedures such as those described by U.S. Pat. No. 4,329,453.

If desired, the crystalline blocked ceftazidime dihydrobromide can also be obtained by adding 48% hydrobromic acid to a solution of the blocked ceftazidime in a suitable solvent such as a halogneated hydrocarbon,

*e.g.*, methylene chloride. In this method, the blocked ceftazidime is extracted with methylene chloride from the acylation mixture described earlier, the extract washed and dried, and then treated with sufficient 48% hydrobromic acid to form the di-salt.

The crystalline salt of this invention is formed and used most advantageously in the overall synthesis of ceftazidime pentahydrate as described earlier. The dihydrobromide salt is a particularly useful form for the isolation and conversion of the blocked ceftazidime and surprisingly is formed without significant concurrent deblocking of the intermediate.

The following non-limiting Examples further illustrate the present invention.

## Example 1

Preparation of crystalline blocked ceftazidime dihydrobromide salt from acylation mixture.

Phosphorus pentachloride (8.74 g) was dissolved in 120 ml of methylene chloride, the solution cooled to -10°C, and 20.2 g (33.72 mmole) of 2-(2-tritylaminothiazol-4-yl)-2-(2-t-butyloxycarbonylprop-2-oxyimino)acetic acid was added. The solution was stirred for 30 min. at -10°C and a cold (0°C) solution of 12 ml (84.3 mmole) of triethylamine in 80 ml of water was added. The two-phase mixture was stirred vigorously for 3 min. The lower phase containing the acid chloride was separated and added over 5 min. to a cold (-10°C) suspension of 11.13 g (28.1 mmole) of 7-amino-3-(1-pyri-

diniummethyl)-3-cephem-4-carboxylic acid dihydrochloride salt in 88 ml of dimethylacetamide containing 20 ml of triethylamine. The temperature of the acylation mixture rose to 5°C during the addition and became thick with suspended solids. After stirring the mixture for 100 min. at 0 to -5°C, 200 ml of deionized water containing 6.2 ml of 48% HBr were added. The two-phase mixture was stirred vigorously until all solids had dissolved. The organic layer was separated and then diluted with 300 ml of isopropyl acetate and the solution treated by dropwise addition with 9.28 ml of 48% HBr. After about one-half of the acid had been added, the solution became hazy and crystallization began. The slurry was stirred for one hour at room temperature and one hour at 0° to 5°C. and then filtered. The crystalline blocked ceftazidime dihydrobromide was washed thoroughly with cold isopropyl acetate and dried overnight at room temperature under vacuum. There were obtained 36.80 g of the dihydrobromide salt as a yellowish tan solid contaminated with a minor amount of an HBr-DMAC complex.

## Example 2

Following the acylation procedure described in Example 1 wherein the same quantities of reactants, reagents, and solvents were used under the same conditions, the acylation to form blocked ceftazidime was repeated. About 200 ml of deionized water containing 6.2 ml of 48% HBr was added to the mixture with stirring. The organic layer was separated and diluted with 300 ml of acetone. Next, about 9.3 ml of

48% HBr was added dropwise. Crystallization began shortly and the resulting slurry was stirred for one hour at room temperature and one hour at 0°C. The crystalline dihydrobromide was filtered, washed with cold acetone, and dried at room temperature for about 12 hours under vacuum. There were obtained 21 g of the di-salt monohydrate as an off white crystalline powder.

## Example 3

The acylation procedure of Example 1 was repeated by using the same quantities of reactants, solvents, and the same conditions and by substituting methyl ethyl ketone for the isopropyl acetate as the counter solvent. There were obtained 25.76 g of crystalline blocked ceftazidime dihydrobromide monohydrate.

Blocked ceftazidime dihydrobromide has the following NMR spectrum: NMR (dmso-$d_6$): δ 7.32 (s, $(C_6H_5)_3$-); 6.83 (s, thiazolyl); 9.11, 8.71, 8.22 (pyridinium); 5.87 (m, C-7 proton); 5.23 (d, C-6 proton); 1.42 (s, $(CH_3)_2C$-); 1.34 (s, *t*-butyl).

## Example 4

Deblocking of (6R,7R)-7-[(Z)-2-(2-tritylaminothiazol-4-yl)-2-(2-t-butyloxycarbonylprop-2-oxyimino)-acetamido]-3-(1-pyridiniummethyl)-3-cephem-4-carboxylic acid dihydrobromide salt.

The dihydrobromide salt of the blocked intermediate, 28.07 g (20.1 mmole), was added slowly with

stirring as a fine crystalline powder to 40 ml of 98% formic acid cooled to 13°C. When no exotherm was observed, the cooling bath was removed. After stirring for 30 min. a clear, dark orange solution was obtained. Trityl alcohol began to crystallize from the solution. After stirring a total of 2 hours at room temperature, the mixture contained (HPLC) about 10% of the starting material, 64% of the detrityl t-butyl ester and about 25% of ceftazidime.2HBr. The reaction mixture was cooled to 10°C and 11.4 ml of 48% hydrobromic acid were added dropwise with stirring to the cold mixture. The cooling bath was removed and stirring was continued for 2 hours at room temperature. HPLC analysis showed about 96% ceftazidime was present.

The mixture was recooled to 10°C and 75 ml of methyl ethyl ketone were added to obtain a solution. The solution was added dropwise over 40 min. to 340 ml of cold methyl ethyl ketone and after addition, the solution was stirred at 0°C for 1 hour.

The deblocked product, ceftazidime dihydrobromide, crystallized, was filtered, washed with 100 ml of cold methyl ethyl ketone, and dried overnight under vacuum at room temperature. There were obtained 11.90 g of the product (corrected yield, 55.7% via HPLC).

## CLAIMS

1. Crystalline (6R,7R)-7-[(Z)-2-(2-tritylaminothiazol-4-yl)-2-(2-t-butyloxycarbonylprop-2-oxyimino)acetamido]-3-(1-pyridiniummethyl)-3-cephem-4-carboxylic acid dihydrobromide.

2. Crystalline (6R,7R)-7-[(Z)-2-(2-tritylaminothiazol-4-yl)-2-(2-t-butyloxycarbonylprop-2-oxyimino)acetamido]-3-(1-pyridiniummethyl)-3-cephem-4-carboxylic acid dihydrobromide monohydrate.

3. A process for preparing crystalline (6R,7R)-7-[(Z)-2-(2-tritylaminothiazol-4-yl)-2-(2-t-butyloxycarbonylprop-2-oxyimino)acetamido]-3-(1-pyridiniummethyl)-3-cephem-4-carboxylic acid dihydrobromide which comprises adding, in the presence of a counter solvent, aqueous hydrobromic acid to (6R,7R)-7-[(Z)-2-(2-tritylaminothiazol-4-yl)-2-(2-t-butyloxycarbonylprop-2-oxyimino)acetamido]-3-(1-pyridiniummethyl)-3-cephem-4-carboxylate.

4. A process as claimed in claim 3 in which the (6R,7R)-7-[(Z)-2-(2-tritylaminothiazol-4-yl)-2-(2-t-butyloxycarbonylprop-2-oxyimino)acetamido]-3-(1-pyridiniummethyl)-3-cephem-4-carboxylate is prepared in situ.

5. A process as claimed in claim 4 in which the (6R,7R)-7-[(Z)-2-(2-tritylaminothiazol-4-yl)-2-(2-t-butyloxycarbonylprop-2-oxyimino)acetamido]-3-(1-pyridiniummethyl)-3-cephem-4-carboxylate is prepared by reacting 7-amino-3-(1-pyridiniummethyl)-3-cephem-4-carboxylate with 2-(2-tritylaminothiazol-4-yl)-2-(2-t-butyloxycarbonylprop-2-oxyimino)acetyl halide.

6. A process as claimed in any one of claims 3 to 5 in which the counter solvent is isopropyl acetate, acetone or methyl ethyl ketone.

7. A process for preparing ceftazidime pentahydrate which comprises preparing blocked ceftazidime dihydrobromide as claimed in claim 1 or 2 and converting it to ceftazidime pentahydrate.

## CLAIMS

1. A process for preparing crystalline (6R,7R)-7-[(Z)-2-(2-tritylaminothiazol-4-yl)-2-(2-t-butyloxycarbonylprop-2-oxyimino)acetamido]-3-(1-pyridiniummethyl)-3-cephem-4-carboxylic acid dihydrobromide which comprises adding, in the presence of a counter solvent, aqueous hydrobromic acid to (6R,7R)-7-[(Z)-2-(2-tritylaminothiazol-4-yl)-2-(2-t-butyloxycarbonylprop-2-oxyimino)acetamido]-3-(1-pyridiniummethyl)-3-cephem-4-carboxylate.

2. A process as claimed in claim 1 in which the (6R,7R)-7-[(Z)-2-(2-tritylaminothiazol-4-yl)-2-(2-t-butyloxycarbonylprop-2-oxyimino)acetamido]-3-(1-pyridiniummethyl)-3-cephem-4-carboxylate is prepared *in situ*.

3. A process as claimed in claim 2 in which the (6R,7R)-7-[(Z)-2-(2-tritylaminothiazol-4-yl)-2-(2-t-butyloxycarbonylprop-2-oxyimino)acetamido]-3-(1-pyridiniummethyl)-3-cephem-4-carboxylate is prepared by reacting 7-amino-3-(1-pyridiniummethyl)-3-cephem-4-carboxylate with 2-(2-tritylaminothiazol-4-yl)-2-(2-t-butyloxycarbonylprop-2-oxyimino)acetyl halide.

4. A process as claimed in any one of claims 1 to 3 in which the counter solvent is isopropyl acetate, acetone or methyl ethyl ketone.

5. A process for preparing ceftazidime pentahydrate which comprises preparing blocked ceftazidime dihydrobromide as defined in claim 1 and converting it to ceftazidime pentahydrate.